# EUROPEAN PATENT SPECIFICATION

(11) **EP 4 098 212 B1**
(45) Date of publication and mention of the grant of the patent: **25.09.2024**
(21) Application number: 22175624.0
(22) Date of filing: 26.05.2022
(51) Int. Cl.: A61B 17/221, A61B 18/14, A61B 90/00, A61B 18/00, A61B 17/22

(54) **MEDICAL TREATMENT SYSTEM**
MEDIZINISCHES BEHANDLUNGSSYSTEM
SYSTÈME DE TRAITEMENT MÉDICAL

(30) Priority: 02.06.2021 US 202117303591
(43) Date of publication of application: 07.12.2022
(73) Proprietor: Covidien LP, Mansfield, MA 02048 (US)
(72) Inventor: Girdhar, Gaurav, Irvine, 92617 (US); Nguyen, Hoai, Irvine, 92617 (US)
(74) Representative: Gray, James

(56) References cited:
- WO-A1-2018/127796
- WO-A1-2019/118321
- US-A1- 2011 301 594
- US-A1- 2014 277 013
- US-A1- 2020 390 456

## Description

### TECHNICAL FIELD

The present technology relates to relates generally to devices, systems, and methods for removing obstructions from body lumens. Some embodiments of the present technology relate to devices and methods for delivering electrical current within a medical treatment system.

### BACKGROUND

Many medical procedures use medical devices to remove an obstruction (such as clot material) from a body lumen, vessel, or other organ. An inherent risk in such procedures is that mobilizing or otherwise disturbing the obstruction can potentially create further harm if the obstruction or a fragment thereof dislodges from the retrieval device. If all or a portion of the obstruction breaks free from the device and flows downstream, it is highly likely that the free material will become trapped in smaller and more tortuous anatomy. In many cases, the physician will no longer be able to use the same retrieval device to again remove the obstruction because the device may be too large and/or immobile to move the device to the site of the new obstruction.

Procedures for treating ischemic stroke by restoring flow within the cerebral vasculature are subject to the above concerns. The brain relies on its arteries and veins to supply oxygenated blood from the heart and lungs and to remove carbon dioxide and cellular waste from brain tissue. Blockages that interfere with this blood supply eventually cause the brain tissue to stop functioning. If the disruption in blood occurs for a sufficient amount of time, the continued lack of nutrients and oxygen causes irreversible cell death. Accordingly, it is desirable to provide immediate medical treatment of an ischemic stroke.

To access the cerebral vasculature, a physician typically advances a catheter from a remote part of the body (typically a leg) through the abdominal vasculature and into the cerebral region of the vasculature. Once within the cerebral vasculature, the physician deploys a device for retrieval of the obstruction causing the blockage. Concerns about dislodged obstructions or the migration of dislodged fragments increases the duration of the procedure at a time when restoration of blood flow is paramount. Furthermore, a physician might be unaware of one or more fragments that dislodge from the initial obstruction and cause blockage of smaller more distal vessels.

Many physicians currently perform thrombectomies (i.e. clot removal) with stents to resolve ischemic stroke. Typically, the physician deploys a stent into the clot in an attempt to push the clot to the side of the vessel and re-establish blood flow. Tissue plasminogen activator ("tPA") is often injected into the bloodstream through an intravenous line to break down a clot. However, it takes time for the tPA to reach the clot because the tPA must travel through the vasculature and only begins to break up the clot once it reaches the clot material. tPA is also often administered to supplement the effectiveness of the stent. Yet, if attempts at clot dissolution are ineffective or incomplete, the physician can attempt to remove the stent while it is expanded against or enmeshed within the clot. In doing so, the physician must effectively drag the clot through the vasculature, in a proximal direction, into a guide catheter located within vessels in the patient's neck (typically the carotid artery). While this procedure has been shown to be effective in the clinic and easy for the physician to perform, there remain some distinct disadvantages to using this approach.

For example, one disadvantage is that the stent may not sufficiently retain the clot as it pulls the clot to the catheter. In such a case, some or all of the clot might remain in the vasculature. Another risk is that, as the stent mobilizes the clot from the original blockage site, the clot might not adhere to the stent as the stent is withdrawn toward the catheter. This is a particular risk when passing through bifurcations and tortuous anatomy. Furthermore, blood flow can carry the clot (or fragments of the clot) into a branching vessel at a bifurcation. If the clot is successfully brought to the end of the guide catheter in the carotid artery, yet another risk is that the clot may be "stripped" or "sheared" from the stent as the stent enters the guide catheter.

In view of the above, there remains a need for improved devices and methods that can remove occlusions from body lumens and/or vessels.

US2020/390456 A1 describes a distal element comprising an expandable mesh. a treatment device includes an elongated member having a proximal portion and a distal portion configured to be positioned within a blood vessel at a treatment site at or near a thrombus.

WO2019/118321 A1 describes an electrically enhancing attachment of the material to the removal device. The removal device can have a core assembly that includes a hypotube coupled to a first electrical terminal and a pushwire coupled to a second electrical terminal, the pushwire extending through the hypotube lumen.

US2014/277013 A1 describes an obstruction removal device is described having a retrieval component used to engage an obstruction within the vasculature and a sheath component that is capable of inverting to fold over the obstruction and the retrieval component. The sheath component helps contain the obstruction and minimizes trauma to the blood vessel during the removal process.

US2011/301594 A1 describes a flexible catheter device capable of being introduced into body passages, withdraw fluids therefrom or introduce fluids thereinto, and which includes electrodes configured to apply electrical signals in the body passage for carrying out thrombus dissolution and/or thrombectomy, wherein one of said electrodes is designed to contact the thrombus material and remove it or dissolve it, and wherein the electrical voltage signals are a unipolar pulsatile voltage signal.

WO 2018/127796 A1 describes an apparatus (21) for removal of a thrombus from a body of a subject. The apparatus includes a first electrode (3), made of a first conductive metal having a first electronegativity, and a second electrode (26), made of a second conductive metal having a second electronegativity that is less than the first electronegativity.

### SUMMARY

The invention is defined in claim 1. Further embodiments are defined in the dependent claims.

Mechanical thrombectomy (e.g., clot-grabbing and removal) has been effectively used for treatment of ischemic stroke. Although most clots can be retrieved in a single pass attempt, there are instances in which multiple attempts are needed to fully retrieve the clot and restore blood flow through the vessel. Additionally, there exist complications due to detachment of the clot from the interventional element during the retrieval process as the interventional element and clot traverse through tortuous intracranial vascular anatomy. For example, the detached clot or clot fragments can obstruct other arteries leading to secondary strokes. The failure modes that contribute to clot release during retrieval are: (a) boundary conditions at bifurcations; (b) changes in vessel diameter; and (c) vessel tortuosity, amongst others. Certain blood components, such as platelets and coagulation proteins, display negative electrical charges. The treatment systems of the present technology provide an interventional element and a current generator configured to positively charge the interventional element during one or more stages of a thrombectomy procedure. For example, the current generator may apply a constant or pulsatile direct current (DC) to the interventional element. The positively charged interventional element attracts negatively charged blood components, thereby improving attachment of the thrombus to the interventional element and reducing the number of device passes or attempts necessary to fully retrieve the clot. In some aspects of the present technology, the treatment system includes an elongate core assembly (e.g., a cable) extending between the current generator and the interventional element. A delivery electrode may be integrated into the core assembly and/or interventional element, and the treatment system further includes a negative electrode that may be disposed at a number of different locations. For example, the negative electrode can be a wire coupled to or integrated within the core assembly. Additionally or alternatively, a negative electrode can take the form of a needle, a grounding pad, a conductive element carried by a one or more catheters of the treatment system, a separate guide wire, and/or any other suitable conductive element configured to complete an electrical circuit with the delivery electrode and the extracorporeally positioned current generator. When the interventional element is placed in the presence of blood (or any other electrolytic medium) and voltage is applied at the terminals of the current generator, current flows along the core assembly to the interventional element, through the blood, and to the return electrode, thereby positively charging at least a portion of the interventional element and adhering clot material thereto.

One approach to delivering current to an interventional element is to conduct current along a core member coupled to a proximal end of the interventional element. However, the inventors have discovered that this approach can lead to disadvantageous concentration of electrical charge along a proximal portion of the interventional element, with insufficient charge density in more distal portions of the interventional element (e.g., along some or all of the working length of the interventional element). This is particularly true of an interventional element having a proximal portion that tapers to a connection point with the core member. This concentration of current in the proximal portion can reduce the efficacy of electrostatic enhancement of clot adhesion, as the mechanical clot engagement occurs primarily at a location distal to the region at which the charge density is greatest. Additionally, when used in an aqueous chloride environment, such as the blood, hydrogen and chlorine gas bubbles can form along the surface of the interventional element in areas with high surface charge density (e.g., along a proximal portion of the interventional element). To reduce risk to the patient and ensure the treatment system functions properly, it may be beneficial to ensure that current flows through the entire interventional element, particularly ensuring sufficient current density in distal portions of the interventional element. When the entire interventional element exhibits a positive electrical charge, all portions of the interventional element can attract negatively charged blood components, thereby improving attachment of the thrombus to the interventional element. If portions of the interventional element are not positively charged (e.g., the distal portion is electrically neutral or exhibits insufficient charge density), those portions of the interventional element may not adequately attract negatively charged blood components, which can prevent improved attachment of the thrombus to the interventional element.

Embodiments of the present technology address these and other problems by providing an electrically conductive coating to one or more components of the treatment system. The conductive coating can be applied to an outer surface of the interventional element. By coating the interventional element with an electrically conductive material, current can easily be distributed through the interventional element instead of concentrating at the more proximal portions of the interventional element. Additionally or alternatively, a conductive coating can be applied to a distal end portion of the core assembly. The core assembly can take the form of an elongated conductor, such as a wire, and be positioned in relation to the interventional element such that the coated distal end portion is positioned distally of the interventional element's distal end. Positioning the interventional element and core assembly in this manner, along with applying a conductive coating to the core assembly and/or interventional element, encourages current to flow through all portions of the interventional element and thereby allows for the interventional element to reliably maintain a positive charge during treatment.

Additional features and advantages of the present technology are described below, and in part will be apparent from the description, or may be learned by practice of the present technology. The advantages of the present technology will be realized and attained by the structure particularly pointed out in the written description and claims hereof as well as the appended drawings. The subject technology is illustrated, for example, according to various aspects described below. These various aspects are provided as examples and do not limit the subject technology.

In one embodiment, a medical device is described. The medical device can include a core assembly configured to be advanced within a corporeal lumen. The core assembly can include a first conductor having a proximal end portion and a distal end portion, the first conductor forming a lumen; a second conductor at least partially disposed within the lumen of the first conductor, the second conductor comprising a first conductive material and having a proximal end portion and a distal end portion extending distal to the distal end portion of the first conductor; an insulative material disposed over at least a portion of the second conductor, the insulative material defining one or more uninsulated portions of the second conductor; and a second conductive material surrounding the second conductor along at least part of the one or more uninsulated portions, the second conductive material having a higher electrical conductivity than the first conductive material. The medical device can also include an interventional element coupled to the distal end portion of the first conductor, the interventional element being electrically coupled to the first conductor, the interventional element comprising a body formed of a third conductive material; and a fourth conductive material disposed over at least a portion of the third conductive material of the interventional element, the fourth conductive material having a higher electrical conductivity than the third conductive material.

In some embodiments, the second conductive material and the fourth conductive material are the same. In some embodiments, the second conductive material and the fourth conductive material each comprises gold. In some embodiments, the distal portion of the second conductor defines a distal tip having an enlarged radial dimension.

In one embodiment, a medical device is described. The medical device can include an elongated shaft having a proximal portion configured to be electrically coupled to a terminal of a current generator, an intermediate portion at least partially covered with an insulative material, and a distal portion, the elongated shaft comprising a first conductive material; an elongated tubular member having a proximal portion configured to be electrically coupled to a current generator, a distal portion, and a lumen receiving the elongated shaft therethrough such that the distal portion of the elongated shaft extends distal to the distal portion of the elongated tubular member; an interventional element coupled to the distal portion of the elongated tubular member, the interventional element at least partially surrounding the distal portion of the elongated shaft, the interventional element comprising a second conductive material; an insulative material disposed over at least a portion of the elongated shaft, the insulative material defining one or more uninsulated portions of the elongated shaft; a third conductive material surrounding the distal portion of the elongated shaft along at least a part of its length, the conductive material being configured to increase the electrical conductivity of the distal portion of the elongated shaft; and a fourth conductive material surrounding the interventional element along at least a part of its length, the fourth conductive material being configured to increase the electrical conductivity of the interventional element.

In some embodiments, the third conductive material has a higher electrical conductivity than the first conductive material. In some embodiments, the fourth conductive material has a higher electrical conductivity than the second conductive material. In some embodiments, the third conductive material is positioned radially adjacent to or distal of the interventional element.

In some embodiments, the third conductive material and the fourth conductive material are the same. In some embodiments, the third conductive material and the fourth conductive material each comprises gold. In one embodiment, a method for delivering an electrical current to a treatment device is described. The method can include inserting a treatment device into a patient, the treatment device comprising: a core assembly comprising: a first conductor having a proximal end portion and a distal end portion, the first conductor forming a lumen; a second conductor at least partially disposed within the lumen of the first conductor, the second conductor comprising a first conductive material and having a proximal end portion and a distal end portion extending distal to the distal end portion of the first conductor; an insulative material disposed over at least a portion of the second conductor, the insulative material defining one or more uninsulated portions of the second conductor; and a second conductive material surrounding the second conductor along at least part of the one or more uninsulated portions, the second conductive material having a higher electrical conductivity than the first conductive material. The treatment device can further include an interventional element coupled to the distal end portion of the first conductor of the core assembly, the interventional element being electrically coupled to the first conductor, the interventional element comprising a body formed of a third conductive material; and a fourth conductive material disposed over at least a portion of the third conductive material of the interventional element, the fourth conductive material having a higher electrical conductivity than the third conductive material. The method can further include positioning the treatment device proximate a thrombus within a lumen of a blood vessel at a treatment site; and delivering an electrical current to the treatment device.

In some embodiments, the second conductive material is positioned radially adjacent to or distal of the interventional element. In some embodiments, the second conductive material and the fourth conductive material are the same. In some embodiments, the second conductive material and the fourth conductive material each comprises gold. In some embodiments, the method further includes ceasing delivery of the electrical current to the treatment device after a time period.

### BRIEF DESCRIPTION OF THE DRAWINGS

Many aspects of the present disclosure can be better understood with reference to the following drawings. The components in the drawings are not necessarily to scale. Instead, emphasis is placed on clearly illustrating the principles of the present disclosure.
FIG. 1A shows a perspective view of an electrically enhanced treatment system for retrieving material from a body lumen, in accordance with one or more embodiments of the present technology.
FIG. 1B shows a schematic view of a current generator, in accordance with one or more embodiments of the present technology.
FIG. 1C shows a schematic view of a current generator, in accordance with one or more embodiments of the present technology.
FIG. 1D shows a side schematic view of the treatment device for retrieving material from a body lumen, in accordance with one or more embodiments of the present technology.
FIG. 1E shows an isometric view of an interventional element and an elongate member positioned within a joining element in accordance with one or more embodiments of the present technology.
FIG. 2A shows a plan view of an interventional element in an unfurled configuration in accordance with one or more embodiments of the present technology.
FIG. 2B shows a cross-sectional view of a portion of an interventional element with an insulative material in accordance with one or more embodiments of the present technology.
FIG. 2C shows a cross-sectional view of a portion of an interventional element in accordance with one or more embodiments of the present technology.
FIG. 3A shows a side schematic view of the treatment device for retrieving material from a body lumen in accordance with one or more embodiments of the present technology.
FIG. 3B shows a cross-sectional view of a portion of a conductor with an insulative material in accordance with one or more embodiments of the present technology.
FIG. 3C shows a cross-sectional view of a portion of a conductor in accordance with one or more embodiments of the present technology.
FIG. 4 shows a side schematic view of the treatment device for retrieving material from a body lumen in accordance with one or more embodiments of the present technology.
FIG. 5 shows a side schematic view of the treatment device for retrieving material from a body lumen in accordance with one or more embodiments of the present technology.
FIG. 6 shows a side schematic view of the treatment device for retrieving material from a body lumen in accordance with one or more embodiments of the present technology.

### DETAILED DESCRIPTION

The present technology provides devices, systems, and methods for removing clot material from a blood vessel lumen. Although many of the embodiments are described below with respect to devices, systems, and methods for treating a cerebral or intracranial embolism, other applications and other embodiments, in addition to those described herein, are within the scope of the technology. For example, the treatment systems and methods of the present technology may be used to remove emboli from body lumens other than blood vessels (e.g., the digestive tract, etc.) and/or may be used to remove emboli from blood vessels outside of the brain (e.g., pulmonary, abdominal, cervical, or thoracic blood vessels, or peripheral blood vessels including those within the legs or arms, etc.). In addition, the treatment systems and methods of the present technology may be used to remove luminal obstructions other than clot material (e.g., plaque, resected tissue, foreign material, etc.). In some embodiments, aspects of the present technology can be applied to medical devices and systems that are not configured for removal of material from vessel lumens, for example systems and devices for ablation, neuromodulation, or any other suitable medical procedure.

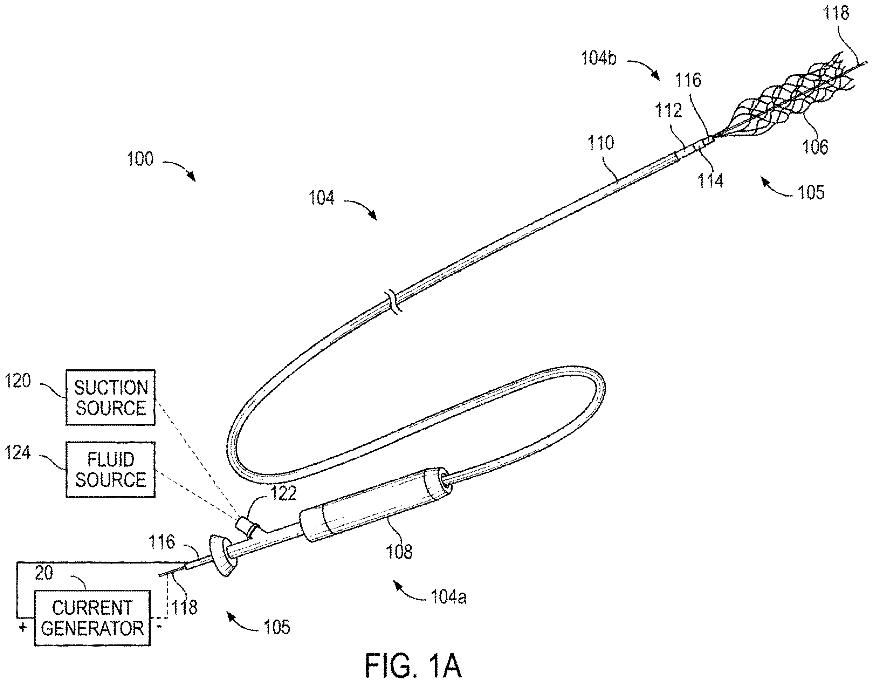

FIG. 1A illustrates a view of an electrically enhanced treatment system 100 according to one or more embodiments of the present technology. As shown in FIG. 1A, the treatment system 100 can include a current generator 20 and a treatment device 104 having a proximal portion 104a configured to be coupled to the current generator 20 and a distal portion 104b configured to be intravascularly positioned within a blood vessel (such as an intracranial blood vessel) at a treatment site at or proximate a thrombus. The treatment device 104 includes an interventional element 106 at the distal portion 104b, a handle 108 at the proximal portion 104a, and a plurality of elongated shafts or members extending therebetween. For example, in some embodiments, such as that shown in FIG. 1A, the treatment device 104 includes a first catheter 110 (such as a guide catheter or balloon guide catheter), a second catheter 112 (such as a distal access catheter or aspiration catheter) configured to be slidably disposed within a lumen of the first catheter 110, and a third catheter 114 (such as a microcatheter) configured to be slidably disposed within a lumen of the second catheter 112. In some embodiments, the treatment device 104 can include a core assembly 105 extending between the proximal portion 104a and distal portion 104b of the treatment device 104. In some embodiments, the treatment device 104 does not include the first catheter 110 and/or the second catheter 112.

The core assembly 105 is configured to be slidably disposed within the lumen of the third catheter 114. In the illustrated embodiment, the core assembly 105 can take the form of an electrical cable or other such assembly that includes a first electrical conductor 116 and a second electrical conductor 118. As discussed in more detail below, in some instances the first conductor 116 can take the form of an elongated tube (e.g., a hypotube) made of or including an electrically conductive material, and the second conductor 118 can take the form of an elongated wire or rod that is made of or includes an electrically conductive material. In some embodiments, the second conductor 118 is configured to be disposed within a lumen of the first conductor 116. In some embodiments, the first conductor 116 and second conductor 118 are coaxial. In various embodiments, the first conductor 116 and second conductor 118 are non-slidably coupled together. The first conductor 116 and second conductor 118 can be sized and configured to be advanced through a corporeal lumen to the treatment site. For example, the first conductor 116 and second conductor 118 can be sized to be positioned proximate a thrombus within a lumen of a blood vessel, such as within a patient's neurovasculature. The first conductor 116 and/or the second conductor 118 can be electrically insulated along at least a portion of their respective lengths. In some embodiments, the first catheter 110 can be coupled to (or incorporate) the handle 108, which provides proximal access to the first conductor 116 and second conductor 118.

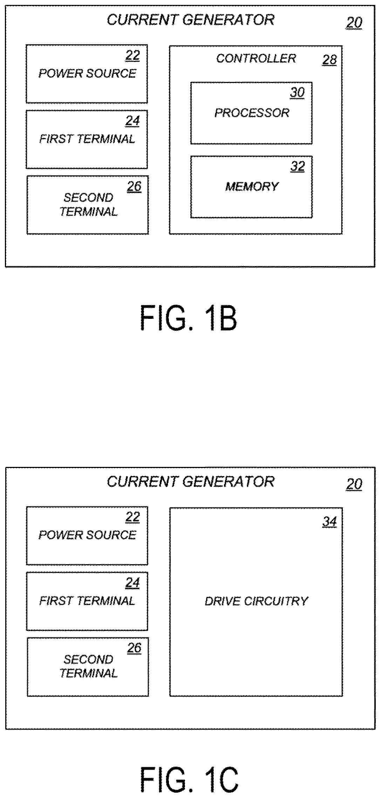

Figures 1B and 1C are schematic views of different embodiments of the current generator 20. With reference to Figure 1B, the current generator 20 can include a power source 22, a first terminal 24, a second terminal 26, and a controller 28. The controller 28 includes a processor 30 coupled to a memory 32 that stores instructions (e.g., in the form of software, code or program instructions executable by the processor or controller) for causing the power source 22 to deliver electric current according to certain parameters provided by the software, code, etc. The power source 22 of the current generator 20 may include a direct current power supply, an alternating current power supply, and/or a power supply switchable between a direct current and an alternating current. The current generator 20 can include a suitable controller that can be used to control various parameters of the energy output by the power source or generator, such as intensity, amplitude, duration, frequency, duty cycle, and polarity. For example, the current generator 20 can provide a voltage of about 2 volts to about 28 volts and a current of about 0.5 mA to about 20 mA.

Figure 1C illustrates another embodiment of the current generator 20, in which the controller 28 of Figure 1B is replaced with drive circuitry 34. In this embodiment, the current generator 20 can include hardwired circuit elements to provide the desired waveform delivery rather than a software-based generator of Figure 1B. The drive circuitry 34 can include, for example, analog circuit elements (e.g., resistors, diodes, switches, etc.) that are configured to cause the power source 22 to deliver electric current via the first and second terminals 24, 26 according to the desired parameters. For example, the drive circuitry 34 can be configured to cause the power source 22 to deliver periodic waveforms via the first and second terminals 24, 26.

The current generator 20 may be coupled to the core assembly 105 to deliver electrical current to the interventional element 106 and thereby provide an electrically charged environment at the distal portion 104b of the treatment device 104. Further, the current generator 20 may be coupled to the core assembly 105 to return electrical current from the electrically charged environment to the current generator 20. In various embodiments, the current generator 20 can be electrically coupled to the first conductor 116, the second conductor 118, or both.

In some embodiments, the treatment system 100 includes a suction source 120 (e.g., a syringe, a pump, etc.) configured to be fluidically coupled (e.g., via a connector 122) to a proximal portion of one or more of the first catheter 110, the second catheter 112, and/or the third catheter 114 to apply negative pressure therethrough. In some embodiments, the treatment system 100 includes a fluid source 124 (e.g., a fluid reservoir, a syringe, pump, etc.) configured to be fluidically coupled (e.g., via the connector 122) to a proximal portion of one or more of the first catheter 110, the second catheter 112, and/or the third catheter 114 to supply fluid (e.g., saline, contrast agents, a drug such as a thrombolytic agent, etc.) to the treatment site.

According to some embodiments, the catheters 110, 112, and 114 can each be formed as a generally tubular member extending along and about a central axis. According to some embodiments, the third catheter 114 is generally constructed to track over a conventional guidewire in the cervical anatomy and into the cerebral vessels associated with the brain and may also be chosen according to several standard designs that are generally available. Accordingly, the third catheter 114 can have a length that is at least 125 cm long, and more particularly may be between about 125 cm and about 175 cm long. Other designs and dimensions are contemplated.

The second catheter 112 can be sized and configured to slidably receive the third catheter 114 therethrough. As noted above, the second catheter 112 can be coupled at a proximal portion to a suction source 120 (FIG. 1A) such as a pump or syringe in order to supply negative pressure to a treatment site. The first catheter 110 can be sized and configured to slidably receive both the second catheter 112 and the third catheter 114 therethrough. In some embodiments, the first catheter 110 is a balloon guide catheter having an inflatable balloon or other expandable member surrounding the catheter shaft at or near its distal end. In operation the first catheter 110 can first be advanced through a vessel and then its balloon can be expanded to anchor the first catheter 110 in place and/or arrest blood flow from areas proximal of the balloon, e.g., to enhance the effectiveness of aspiration performed via the first catheter 110 and/or other catheter(s). Alternatively, a guide catheter without a balloon can be employed. Next, the second catheter 112 can be advanced through the first catheter 110 until its distal end extends distally beyond the distal end of the first catheter 110. The second catheter 112 can be positioned such that its distal end is adjacent or proximal of a treatment site (e.g., a site of a blood clot within the vessel). The third catheter 114 may then be advanced through the second catheter 112 until its distal end extends distally beyond the distal end of the second catheter 112. The interventional element 106 may then be advanced through the third catheter 114 via the core assembly 105 for delivery to the treatment site.

According to some embodiments, the bodies of the catheters 110, 112, and 114 can be made from various thermoplastics, e.g., polytetrafluoroethylene (PTFE or TEFLON^{®}), fluorinated ethylene propylene (FEP), high-density polyethylene (HDPE), polyether ether ketone (PEEK), etc., which can optionally be lined on the inner surface of the catheters or an adjacent surface with a hydrophilic material such as polyvinylpyrrolidone (PVP) or some other plastic coating. Additionally, either surface can be coated with various combinations of different materials, depending upon the desired results.

In some embodiments, the current generator 20 may be coupled to a proximal portion of the first conductor 116, and/or a proximal portion of the third catheter 114, the second catheter 112, and/or first catheter 110 to provide an electric current to the interventional element 106. For example, as shown in FIG. 1A, the current generator 20 can be coupled to a proximal portion of the core assembly 105 and/or the first conductor 116 such that the first conductor 116 functions as a first conductive path (e.g., as a positive conductive path transmitting current from the current generator to the treatment site). As shown in FIG. 1A, the current generator 20 can also be coupled to a proximal portion of the second conductor 118 such that the second conductor 118 functions as a second conductive path (e.g., as a negative conductive path transmitting current from the treatment site to the current generator 20). In other embodiments, the negative electrode can be separate from the second conductor 118. In some embodiments, the positive electrode can comprise the interventional element 106, and the negative electrode can be carried by one or more of the third catheter 114, the second catheter 112, and/or first catheter 110, or be coupled to or formed by a portion of the second conductor 118. In some embodiments, the negative electrode can be provided via one or more external electrodes, such as a needle puncturing the patient, or a grounding pad applied to the patient's skin; in some such embodiments, or otherwise, the first conductor 116 or the second conductor 118 may be omitted from the core assembly 105.

The system can include multiple (e.g., two or more), distinct conductive paths or channels for passing electrical current along the system. The interventional element 106 can serve as one electrode (e.g., a positive electrode) in electrical communication with a conductive path via the first conductor 116. Another of the conductive paths of the system can be in electrical communication with another electrode (e.g., a negative electrode). For example, the second conductor 118 can serve as the second conductive path, with one or more uninsulated portions of the second conductor 118 forming the negative electrode(s).

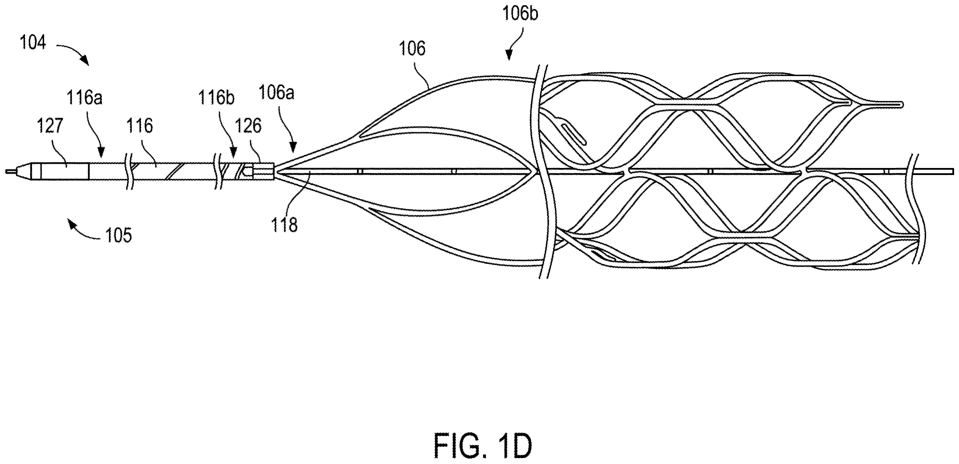

As shown in FIG. 1D, the first conductor 116 and the interventional element 106 can be joined at a connection 126 to secure the interventional element 106 relative to the first conductor 116 and to complete an electrical pathway between the elongate first conductor 116 and the interventional element 106. As illustrated in FIG. 1D, the distal end portion of the second conductor 118 is configured to be positioned distal of the distal end portion of the first conductor 116. The interventional element 106 can be metallic or otherwise electrically conductive so that when the interventional element 106 is placed in the presence of blood (or thrombus, and/or any other electrolytic medium which may be present, such as saline) and voltage is applied via the electrical connectors of the current generator 20, current flows from the positive connector of the current generator 20, distally along the first conductor 116 to the interventional element 106 and through the surrounding media (e.g., blood, tissue, thrombus, etc.) before returning proximally along the second conductor 118 to the negative electrical connector of the current generator 20, thereby positively charging at least a portion of the interventional element 106 and promoting clot adhesion.

In certain embodiments, the polarities of the current generator 20 can be switched, so that the negative electrical connector is electrically coupled to the first conductor 116 and the positive electrical connector is electrically coupled to the second conductor 118. This can be advantageous when, for example, attempting to attract predominantly positively charged material to the interventional element 106, or when attempting to break up a clot rather than grasp it with an interventional element 106. In some embodiments alternating current (AC) signals may be used rather than DC. In certain instances, AC signals may advantageously help break apart a thrombus or other material.

In various embodiments, the interventional element 106 can take any number of forms, for example a removal device, a thrombectomy device, or other suitable medical device. For example, in some embodiments the interventional element 106 may be a stent and/or stent retriever, such as Medtronic's Solitaire^{™} Revascularization Device, Stryker Neurovascular's Trevo^{®} ProVue^{™} Stentriever, or other suitable devices. In some embodiments, the interventional element 106 may be a coiled wire, a weave, and/or a braid formed of a plurality of braided filaments. Examples of suitable interventional elements 106 include any of those disclosed in U.S. Patent No. 7,300,458, filed November 5, 2007, U.S. Patent No. 8,940,003, filed November 22, 2010, U.S. Patent No. 9,039,749, filed October 1, 2010, and U.S. Patent No. 8,066,757, filed December 28, 2010.

The interventional element 106 can have a low-profile, constrained or compressed configuration for intravascular delivery to the treatment site within the third catheter 114, and an expanded configuration for securing and/or engaging clot material and/or for restoring blood flow at the treatment site. The interventional element 106 has a proximal portion including an attachment portion 106a that may be coupled to the first conductor 116 and a distal portion comprising an open cell framework or body 106b. In some embodiments, the body 106b of the interventional element 106 can be generally tubular (e.g., cylindrical), and the proximal portion of the interventional element 106 can taper proximally to the attachment portion 106a. In various embodiments, the interventional element 106 can define a lumen that is located radially inward from the body 106b.

The interventional element 106 can be a metallic and/or electrically conductive thrombectomy device. For example, the interventional element can include or be made of stainless steel, nitinol, cobalt-chromium, platinum, tantalum, alloys thereof, or any other suitable material. In some embodiments, the interventional element 106 is a mesh structure (e.g., a braid, a stent, etc.) formed of a superelastic material (e.g., Nitinol) or other resilient or self-expanding material configured to self-expand when released from the third catheter 114. The mesh structure may include a plurality of struts and open spaces between the struts. In some embodiments, the struts and spaces may be situated along the longitudinal direction of the interventional element 106, the radial direction, or both.

In some embodiments, the first conductor 116 can be a structural element configured to push and pull a device such as the interventional element 106 along the bodily lumen. The first conductor 116 can be any suitable elongate member configured to advance the interventional element 106 to a treatment site within a blood vessel. For example, the first conductor 116 can be or include a wire, tube (e.g., a hypotube), coil, or any combination thereof. According to some embodiments, the first conductor 116 comprises an elongate tubular member defining a lumen therethrough. In some embodiments, the first conductor 116 can comprise a distally located aperture configured to receive the attachment portion of the interventional element. In some embodiments, the first conductor 116 comprises a distally located joining element comprising the aperture configured to receive the attachment portion. The first conductor 116 can have a length sufficient to extend from a location outside the patient's body through the vasculature to a treatment site within the patient's body. The first conductor 116 can be a monolithic structure or formed of multiple joined segments. In some embodiments, the first conductor 116 can comprise a laser-cut hypotube having a spiral cut pattern (or other pattern of cut voids) formed in its sidewall along at least a portion of its length. The first conductor 116 can be metallic and/or otherwise electrically conductive to deliver current from the current generator 20 to the interventional element 106. For example, the first conductor 116 can comprise or consist of nickel titanium alloy, stainless steel, or other metals or alloys. In embodiments that comprise multiple joined segments, the segments may be of the same or different materials. For example, some or all of the first conductor 116 can be formed of stainless steel, or other suitable materials known to those skilled in the art. Nickel titanium alloy may be preferable for kink resistance and reduction of imaging artifacts.

In some embodiments, the second conductor 118 can be a structural element configured to secure or retain a position of the interventional element 106 relative to the first conductor 116. Additionally, or alternatively, the second conductor 118 can be configured to be a negative electrode. The second conductor 118 can be any suitable elongate member configured to extend through a lumen of the first conductor 116. For example, the second conductor 118 can be or include a wire, tube (e.g., a hypotube), coil, or any combination thereof. The second conductor 118 can have a length sufficient to extend from a location outside the patient's body through the vasculature to a treatment site within the patient's body. The second conductor 118 can be a monolithic structure or formed of multiple joined segments. The second conductor 118 can be metallic or electrically conductive to deliver current from the surrounding media (e.g., blood, tissue, thrombus, etc.) to the current generator 200. For example, the second conductor 118 can comprise or consist of nickel titanium alloy, stainless steel, or other metals or alloys. In embodiments that comprise multiple joined segments, the segments may be of the same or different materials. For example, some or all of the second conductor 118 can be formed of stainless steel, or other suitable materials known to those skilled in the art. Nickel titanium alloy may be preferable for kink resistance and reduction of imaging artifacts. The second conductor 118 can be electrically insulated along some or all of its length. In some embodiments, the second conductor 118 comprises an insulated wire or guide wire having one or more exposed, electrically conductive portions. For example, a distal end portion of the second conductor 118 can be exposed to conduct current from surrounding media (e.g., blood, tissue, thrombus, etc.) at a treatment site.

In some embodiments, the treatment device 104 can comprise one or more electrically insulating materials. For example, an insulating material can be disposed on one or more portions of the second conductor 118 to electrically isolate the second conductor 118 from the first conductor 116, the connection 126, and/or the interventional element 106. Additionally or alternatively, an insulating material can be disposed within a lumen of the first conductor 116 to electrically isolate the first conductor 116 from the second conductor 118 and/or the attachment portion of the interventional element 106. In various embodiments, an insulating material is disposed over an outer surface of the interventional element 106 along at least a portion of the length of the interventional element 106. In some embodiments, an insulating material is disposed over an outer surface of the first conductor 116 along at least a portion of a length of the first conductor 116 to direct current through the first conductor 116 and prevent current loss from the first conductor 116 to the surrounding environment. As shown in FIG. 1D, in some embodiments, an insulating material 127 can be disposed adjacent to a proximal end portion 116a and/or a distal end portion 116b of the first conductor 116. The insulating material 127 may be disposed along an entire length of the first conductor 116 and/or the second conductor 118 or the insulating material may be disposed along select portions of the first conductor 116 and/or the second conductor 118. The insulating material 127 may comprise a polymer, such as polyimide, parylene, PTFE, or another suitable electrically insulating material.

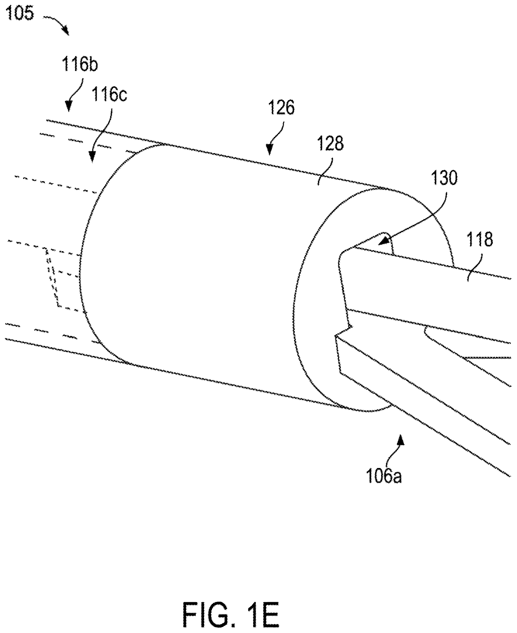

As shown in FIGS. 1D and 1E, the interventional element 106 and the first conductor 116 can be coupled at a connection 126. According to some embodiments, the interventional element 106 and the first conductor 116 can be substantially permanently attached together at the connection 126. That is, the interventional element 106 and the first conductor 116 can be attached together in a manner that, under the expected use conditions of the device, the interventional element 106 and the first conductor 116 would not become unintentionally separated from one another. In some embodiments, the treatment device 104 can comprise a portion, located proximally or distally of the connection 126, that is configured for selective detachment of the interventional element 106 from the first conductor 116. For example, such a portion can comprise an electrolytically severable segment of the first conductor 116. In some embodiments, the device can be devoid of any feature that would permit selective detachment of the interventional element 106 from the first conductor 116. The connection 126 can provide a mechanical interlock between the interventional element 106 and the first conductor 116. Moreover, the connection 126 can be configured to complete an electrically conductive path between the interventional element 106 and the elongate first conductor 116.

FIG. 1E illustrates an enlarged perspective view of the connection 126, according to some embodiments, between the first conductor 116 and the interventional element 106. In some embodiments, for example as shown in FIG. 1E, the first conductor 116 comprises a distally located joining element 128 including an aperture 130 configured to receive a proximally located attachment portion 106a of the interventional element 106 and/or at least a portion of the second conductor 118. As shown in FIG. 1E, the attachment portion 106a of the interventional element 106 is configured to mechanically interlock with a joining element 128 to secure the interventional element 106 to the core assembly 105. In some embodiments, the second conductor 118 can be disposed within the aperture at a radially adjacent position relative to the attachment portion 106a to facilitate such securement. Further, the second conductor 118 may be affixed to the joining element 128 via a weld, an adhesive, a threaded connection, an interference fit, or any other suitable connection.

In some embodiments, the connection 126 can comprise a bonding agent in addition or alternative to the joining element 128 and/or second conductor 118. The bonding agent can comprise adhesive, solder, welding flux, brazing filler, etc., disposed within the joining element 128, and/or adjacent to it, just proximal of and/or just distal of the joining element 128. In some embodiments, the bonding agent can bond to the connection 126 without applying heat. For example, the bonding agent can comprise a UV-curable adhesive. In embodiments that comprise a polymer coating of the wire or polymer tubing, use of a bonding agent that avoids application of heat that would damage the polymer may be preferred.

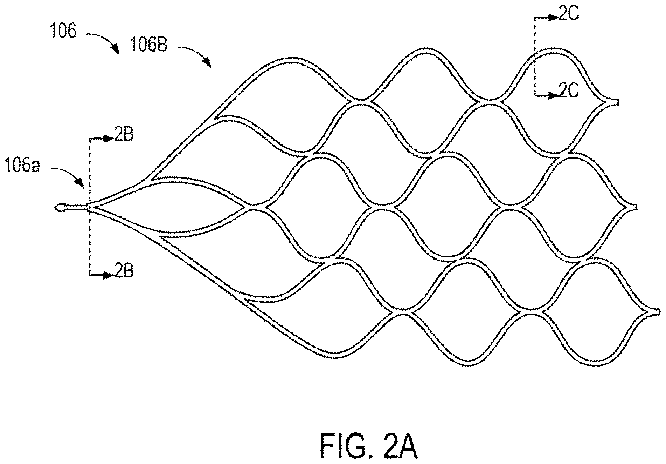

FIG. 2A is a plan view of the interventional element 106, depicted in an unfurled or flattened configuration for ease of understanding. The interventional element 106 has a proximal portion that may be coupled to the first conductor 116 and a distal portion. The interventional element 106 has a proximal portion including an attachment portion 106a that may be coupled to the first conductor 116 and a distal portion comprising an open cell framework or body 106b. The attachment portion 106a of the interventional element 106 can have a substantially constant thickness, such as would result from the interventional element 106 being cut from a tube or sheet of material, for example. In other embodiments, the thickness of the attachment portion 106a can vary across its length, width, or both.

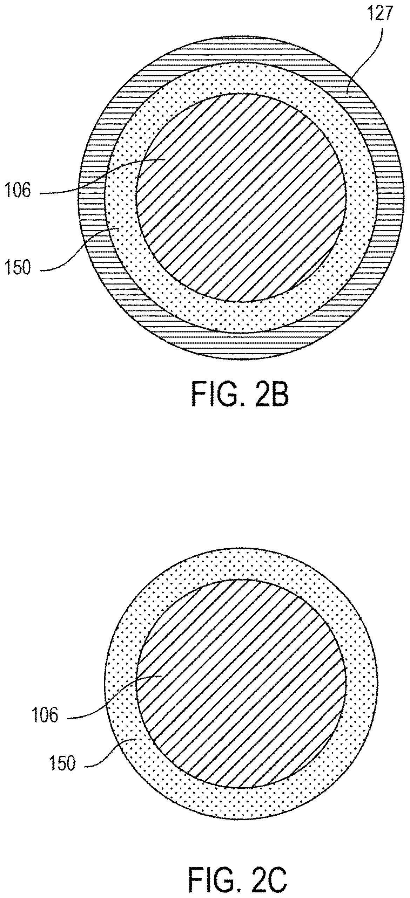

FIGS. 2B and 2C illustrate two cross-sectional views of the interventional element 106 along the lines 2B and 2C in FIG. 2A, respectively. As illustrated in FIG. 2B, the interventional element 106 can include an insulating material 127 and a conductive material 150 coupled to the interventional element 106. The conductive material 150 can be disposed over an outer surface of the interventional element 106 along at least a portion of the length of the interventional element 106. The insulating material 127 can be disposed over an outer surface of the conductive material 150 so that the conductive material 150 is radially disposed between the interventional element 106 and the insulating material 127. In some embodiments, one or more portions of the interventional element 106 are uninsulated, which exposes the underlying conductive material 150. For example, as illustrated in FIG. 2C, the outer surface of the conductive material 150 is uninsulated, exposing the conductive material 150. In various embodiments, one or more portions of the interventional element 106 do not include a conductive material 150 disposed over the outer surface. For example, some portions of the interventional element 106 can include an insulating material 127 disposed over the outer surface of the body 106b with no intervening conductive material 150 disposed between the insulating material 150 and the body 106b. In some embodiments, the conductive material 150 surrounds substantially all of the interventional element 106. In some embodiments, various sections of the interventional element 106 can be coated with the conductive material 150 and/or the insulating material 127 as shown in FIG. 2B or as shown in FIG. 2C.

The conductive material 150 can increase the electrical conductivity of the interventional element 106. The conductive material 150 can include a material that has a higher electrical conductivity than the material used to form the body of the interventional element 106. For example, the conductive material 150 can be a gold coating while the interventional element body can be formed from Nitinol. By coupling a higher electrically conductive material to the body of the interventional element 106, an electric current can more easily pass through the interventional element 106 via the conductive material 150, which thus, increases the electrical conductivity of the interventional element 106. In some embodiments, the insulative material 127 can electrically isolate one or more portions of the interventional element. For example, the insulated material 127 can electrically isolate the attachment portion 106a of the interventional element 106. By electrically isolating portions of the interventional element 106, the insulating material 127 can be used to prevent electrical shortages between the interventional element 106 and the second conductor 118. Additionally, the electrically isolating portions of the interventional element 106 with insulating material 127 can encourage current to flow to the distal portions of the interventional element 106.

In some embodiments, the conductive material 150 is disposed in a thin layer on the outer surface of the interventional element 106. For example, the conductive material 150 can have a thickness between about 0.05 microns to about 5 microns. Having a thickness within this range allows for the conductive material 150 to distribute current through the interventional element 106 without mechanically impacting the interventional element 106. In some embodiments, the conductive material 150 can be formed from any suitable electrically conductive material. For example, the conductive material 150 can be formed from gold, silver, copper, platinum, palladium, iridium, ruthenium, rhodium, or corresponding alloys and combinations. In various embodiments, the conductive material 150 is formed from a metallic material. Additionally or alternatively, the conductive material 150 is formed from a noble metal. In some embodiments, the conductive material 150 is coupled to the interventional element 106 by coating, plating, surface-treating, or through vapor deposition.

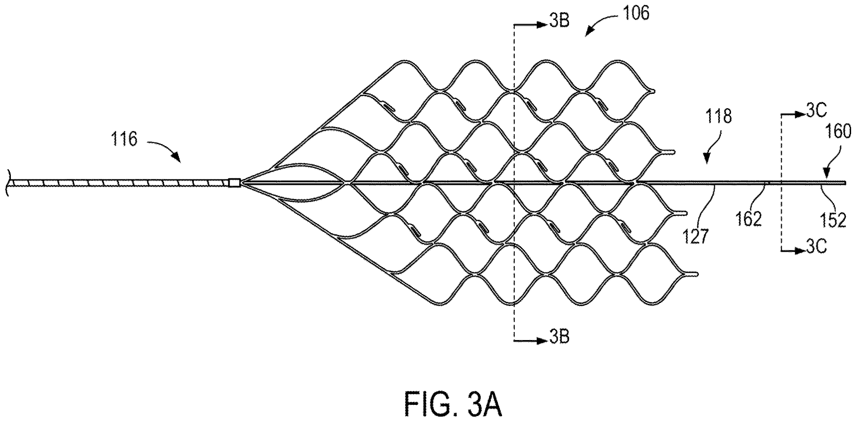

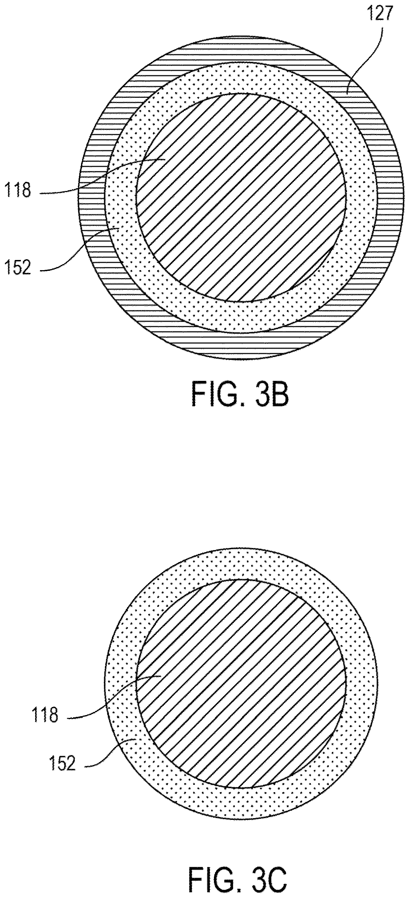

FIG. 3A illustrates a side schematic view of a portion of the treatment device 104. FIGS. 3B and 3C illustrate cross-sectional views of the second conductor 118 along the lines 3B and 3C of FIG. 3A, respectively, with portions of the treatment 104 device hidden for clarity. As illustrated in FIG. 3A, the interventional element 106 is coupled to, and extends distally from, the first conductor 116. The second conductor 118 can extend through the lumen of the first conductor 116 and beyond a distal end of the first conductor 116 such that a distal portion of the second conductor 118 extends through an interior region of the interventional element 106. In some embodiments, the second conductor 118 can define a distal tip 160 at the distal portion 162 of the second conductor. As noted previously, an insulative material 127 can surround second conductor 118 along at least a portion of its length. At one or more positions along a distal region of the second conductor 118, the second conductor 118 can include one or more uninsulated portions. In some embodiments, a conductive material 152 can couple to the one or more uninsulated portions. As illustrated in FIGS. 3B and 3C, the conductive material 152 can be disposed over an outer surface of the second conductor 118 such that the conductive material 152 surrounds the second conductor 118 along at least a part of the length of the second conductor 118. In some embodiments, the conductive material 152 couples to the second conductor 118 only at the uninsulated portions of the second conductor 118. Additionally or alternatively, the conductive material 152 couples to the second conductor 118 along substantially the entire length of the second conductor 118. In various embodiments, the conductive material 152 can be positioned radially adjacent to or distal of the interventional element. In some embodiments, the conductive material 152 can be radially disposed between the insulative material 127 and the second conductor 118. In some embodiments, various sections of the second conductor 118 can be coated with the conductive material 152 and/or the insulating material 127 as shown in FIG. 3B or as shown in FIG. 3C

The conductive material 152 can increase the electrical conductivity of the second conductor 118. The conductive material 152 can comprise a material that has a higher electrical conductivity than the material used to form the body of the second conductor 118. For example, the conductive material 152 can be a gold coating while the body of the second conductor 118 can be formed from stainless steel. By coupling a higher electrically conductive material to the body of the second conductor 118, an electric current can more easily pass through the second conductor 118 via the conductive material 152, which thus, increases the electrical conductivity of the second conductor 118. In some embodiments, the insulative material 127 can electrically isolate one or more portions of the interventional element. For example, the insulated material 127 can electrically isolate the portions of the second conductor 118 that are adjacent the attachment portion 106a of the interventional element 106. By electrically isolating portions of second conductor 118, the insulating material 127 can be used to prevent electrical shortages between the interventional element 106 and the second conductor 118. Additionally, electrically isolating portions of the second conductor 106 with insulating material 127 can encourage current to flow to the desired portions of the interventional element 106 (e.g., more distal portions). For example, as illustrated in FIG. 3A, the insulating material 127 can electrically isolate portions of the second conductor 118 adjacent the interventional element 106 while the distal tip 160 of the second conductor remains uninsulated. In this arrangement, or otherwise, current will be encouraged to flow to the distal portions of the interventional element 106 to reach the uninsulated distal tip 160, as the adjacent portions of the second conductor 118 are electrically insulated.

In some embodiments, the conductive material 152 is disposed in a thin layer on the outer surface of the second conductor 118. For example, the conductive material 152 can have a thickness between about 0.05 microns to about 5 microns. Having a thickness within this range allows for the conductive material 152 to distribute current through the second conductor 118 without mechanically impacting the second conductor 118. In some embodiments, the conductive material 152 can be formed from an electrically conductive material. For example, the conductive material 152 can be formed from gold, silver, copper, platinum, palladium, iridium, ruthenium, rhodium, or corresponding alloys and combinations. In various embodiments, the conductive material 152 is formed from a metallic material. Additionally or alternatively, the conductive material 152 is formed from a noble metal. In some embodiments, the conductive material 152 is substantially the same as the conductive material 150. In various embodiments, the conductive material 152 is coupled to the second conductor 118 by coating, plating, surface-treating, or through vapor deposition.

In various embodiments, the conductive material 152 of the second conductor 118 can be disposed radially adjacent to or distal of interventional element 106. For example, the conductive material 152 can be disposed distally of the connection 126 and be positioned radially inward of the interventional element 106. In some embodiments, one or more conductive material 152 portions extend (or are positioned) distally of the distal end of the interventional element 106. In various embodiments, the second conductor 118 and the conductive material 152 are disposed within the lumen of the interventional element 106. In some embodiments, the conductive material 152 can be disposed proximate or radially adjacent to the spaces or cell openings 107 bounded by the struts of the body 106b of the interventional element 106. In some embodiments, each portion of the second conductor 118 with the conductive material 152 is positioned radially adjacent to a cell opening 107 of the interventional element 106, and/or each portion of the second conductor 118 that is radially adjacent to a strut (and/or other metallic component, such as a radiopaque marker) of the interventional element 106 is insulated.

As noted previously, the interventional element 106 can be in electrical communication with the first conductor 116, such that current supplied to a proximal end of the first conductor 116 is carried through the first conductor 116 to the interventional element 106. The second conductor 118 can be covered with an electrically insulative material 127 with one or more uninsulated portions along the distal region of the second conductor 118. The uninsulated portions can expose the underlying conductive material 152, allowing for second conductor 118 to be electrically coupled to the interventional element 106 at the distal end portion of the second conductor 118. In operation, the current generator 20 can couple to the core assembly 105 at the proximal portion 104a of the treatment device 104 and send a current through the first conductor 116. This current can flow through the first conductor 116 to the interventional element 106 via the conductive material 150. At the interventional element 106, the current can flow through the patient's surrounding media (e.g., blood, tissue, saline, thrombus, etc.) to the uninsulated portions of the second conductor 118 via the conductive material 152, where the current can then flow through the second conductor 118 and return to the current generator 20.

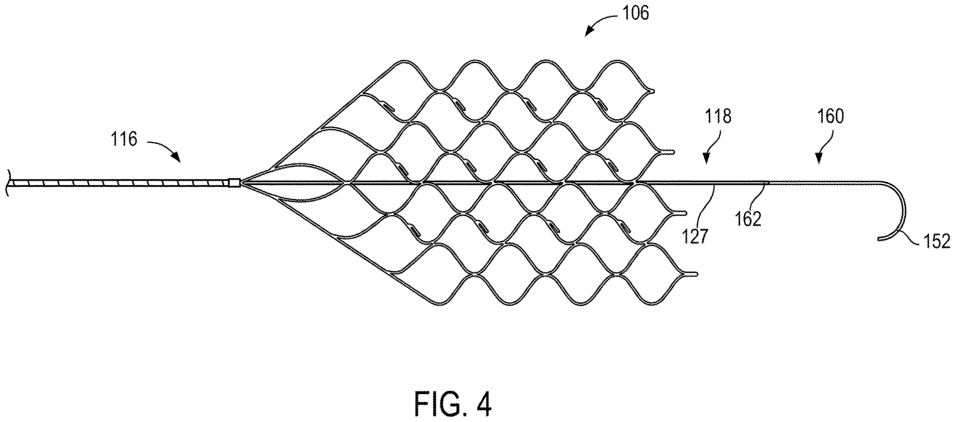

FIGS. 4 to 6 illustrate several schematic side views of a portion of the treatment device 104 according to one or more embodiments of the present technology. As noted previously, in some embodiments, the second conductor 118 can include a distal tip 160. The distal tip 160 can be formed at the distal portion 162 of the second conductor 118 and can be uninsulated.

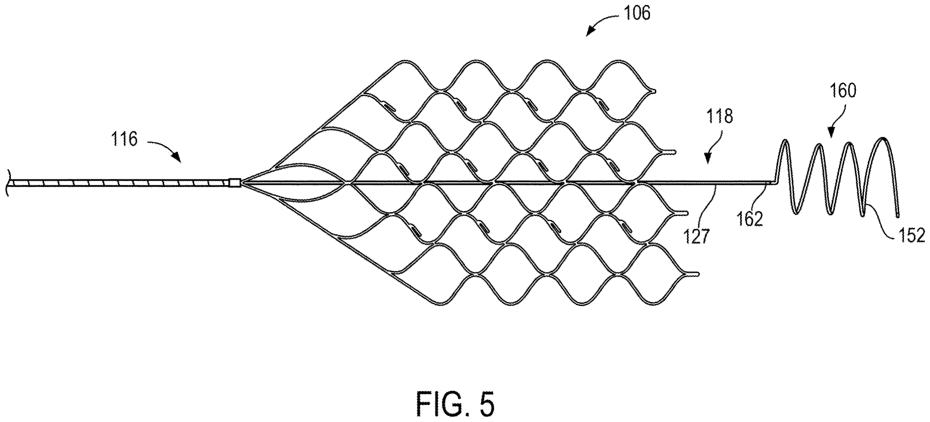

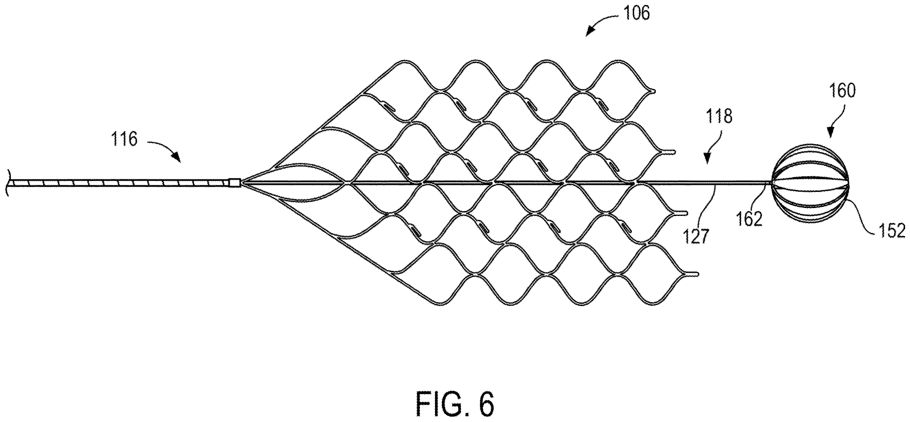

The distal tip 160 can be formed in a variety of different shapes and sizes. For example, as illustrated in FIG. 4, the distal tip 160 can form a "J" shape, where the distal tip 160 has a first portion that extends distally from the distal portion 162 of the second conductor 118, a second portion that curves and extends laterally, and a third portion that extends proximally towards the distal portion 162. In some embodiments, the distal tip 160 can be formed in a helical or spiral shape. For example, as illustrated in FIG. 5, the distal tip 160 can be formed in a helical shape that extends distally from the distal portion 162 of the second conductor 118. In various embodiments, the distal tip 160 can form a spherical or spheroid shape. For example, as illustrated in FIG. 6, the distal tip forms a partially hollowed sphere at the distal portion 162. Additionally or alternatively, the distal tip 160 can have an enlarged radial dimension compared to the second conductor 118. The enlarged radial dimension increases the surface area of the distal tip 160 relative to the second conductor 118. In some embodiments, the distal tip 160 can comprise a distal embolization protection element, for example a basket, mesh, or filter configured to capture any fragments that separate from the thrombus during engagement with the interventional element 106. Although several examples herein refer to a distal tip, in various embodiments such a distal element (e.g., J-shaped wire, helical or spiral winding, spheroid shape, or other radially enlarged portion) at a position spaced proximally from a distal terminus of the second conductor 118. For example, such a distal element may be coupled to the second conductor 118 at a position distal of the interventional element 106, while the second conductor 118 may extend distally beyond such a distal element, such as in the form of a wire or other feature extending distally of the distal element.

The distal tip 160 can improve the surface charge density along the surface of the second conductor 118. For example, the distal tip 160 can have structural features (e.g., curves, spiral shape, an expandable spheroid shape, etc.) that provide an enlarged surface area compared to a straight wire or rod. This enlarged surface area may allow for the charge density to distribute throughout the surface of the distal tip 160 more evenly. By distributing the charge density in this manner, the risk of hydrogen and chlorine gas bubbles forming along the surface of the second conductor 118 is reduced.

In some embodiments, the conductive material 152 can be coupled to the distal tip 160. For example, the conductive material 152 can surround the distal tip 160 along at least a portion of the length of the distal tip 160. In some embodiments, the surface area defined by the conductive material 152 coupled to the distal tip 160 is between about 5% to about 50% of the surface area defined by the conductive material 150 coupled to the interventional element 106. Coupling the conductive material 152 to the distal tip 160 can increase the electrical conductivity of the distal tip 160. The conductive material 152 can comprise a material that has a higher electrical conductivity than the material used to form the distal tip 160. For example, the conductive material 152 can be formed from a gold coating while the distal tip 160 can be formed from stainless steel. By coupling a more electrically conductive material to the distal tip 160, an electric current can more easily pass through the distal tip via the conductive material 152, which thus, increases the electrical conductivity of the distal tip 160.

In various embodiments, distal tip 160 can be configured to allow for the interventional element 106 to maintain a desirable electrical charge distribution. For example, positioning the distal tip 160 proximate the distal end portion of the second conductor 118 encourages the more current to flow through the distal portions of the interventional element 106 to reach the distal tip 160, which in turn allows for the interventional element 106 to maintain a favorable electrical charge distribution (e.g., with sufficiently high charge density at the distal region of the interventional element, along the working length of the interventional element, or other suitable charge distribution).

An example method of delivering a current to the treatment device 104 will now be described. First, the treatment device 104 is positioned within a patient at the treatment site. Once the treatment device 104 is properly positioned, the user can expand the interventional element 106 so that the interventional element 106 engages with the thrombus. After the interventional element 106 engages with the thrombus, the user can couple the core assembly 105 to the current generator 20. In some embodiments, the core assembly 105 is previously coupled to the current generator 20. The user can interact with current generator to initiate the supply of an electrical signal to the first conductor 116. The electrical signal can travel toward the treatment site through the first conductor 116 and to the interventional element 106 via the conductive material 150. The electrical signal can return to the current generator 20 by flowing from the interventional element 106, through the surrounding media (e.g., blood, tissue, thrombus, etc.) to the second conductor 118 via the conductive material 152 and through the second conductor 118 to the current generator 20. In some embodiments, the electrical signal is an electrical current of between about 0-5 mA. The electrical signal can be unipolar (e.g., DC) or bipolar (e.g., AC). In various embodiments, the current or voltage level of the electrical signal can be constant, periodic, irregular, or any combination thereof. In some embodiments, the electrical signal is supplied for a duration of time between about 30 seconds to about 10 minutes. In some embodiments, the electrical signal is supplied for a duration of time of two minutes or less. After the electrical signal is delivered to the treatment device 104 for the proper duration, the user can interact with current generator to stop the supply of the electrical signal. The user can then proximally retract the treatment device 104, including the thrombus, into a surrounding catheter, and then remove the entire assembly from the patient.

### Conclusion

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A medical device (100) comprising:
a core assembly (105) configured to be advanced within a corporeal lumen, the core assembly comprising:
a first conductor (116) having a proximal end portion (116a) and a distal end portion (116b), the first conductor forming a lumen;
a second conductor (118) at least partially disposed within the lumen of the first conductor, the second conductor comprising a first conductive material and having a proximal end portion and a distal end portion extending distal to the distal end portion of the first conductor;
an insulative material (127) disposed over at least a portion of the second conductor, the insulative material defining one or more uninsulated portions of the second conductor; and
a second conductive material (150, 152) surrounding the second conductor along at least part of the one or more uninsulated portions, the second conductive material having a higher electrical conductivity than the first conductive material;
an interventional element (106) coupled to the distal end portion of the first conductor, the interventional element being electrically coupled to the first conductor, the interventional element comprising a body (106b) formed of a third conductive material; and
a fourth conductive material disposed over at least a portion of the third conductive material of the interventional element, the fourth conductive material having a higher electrical conductivity than the third conductive material.

2. The medical device (100) of Claim 1, wherein the second conductive material is positioned radially adjacent to or distal of the interventional element (106).

3. The medical device (100) of any of Claim 1 or Claim 2, wherein the third conductive material comprises Nitinol, and/or the first conductive material comprises stainless steel.

4. The medical device (100) of any of Claims 1 to 3, wherein the distal portion of the second conductor (118) defines a distal tip (160) having an enlarged radial dimension.

5. The medical device (100) of Claim 4, wherein the one or more uninsulated portions comprises the distal tip (160).

6. The medical device (100) of any of the Claims 1 to 5, wherein a surface area defined by the second conductive material is at least about 15% of a surface area defined by the fourth conductive material.

7. The medical device (100) of any of the Claims 1 to 6, wherein the first and second conductors (116, 118) are coaxial.

8. The medical device (100) of any of the Claims 1 to 7, wherein the core assembly (105) comprises a distally located joining element (128) having an opening therein, and wherein the second conductor extends through the opening.

9. The medical device (100) of any of the Claims 1 to 8, wherein the interventional element (106) defines a lumen, and wherein the second conductor (118) extends at least partially through the lumen.

10. The medical device (100) of Claim 9, wherein the second conductive material is disposed within the lumen.

11. The medical device (100) of any of the Claims 1 to 10, wherein the first conductor and the second conductor are non-slidably coupled together.

12. The medical device (100) of any of the Claims 1 to 11, wherein the core assembly (105) is sized and configured to be advanced through a blood vessel to a treatment site.

13. The medical device (100) of any of the Claims 1 to 12, wherein the distal end portion of the second conductor (118) is positioned distal of the distal end portion of the first conductor (116).

14. The medical device (100) of any of the Claims 1 to 13, wherein the interventional element (106) comprises a plurality of struts that are bounded to form a cell opening, the conductive portion being positioned radially adjacent to the cell opening.

15. The medical device (100) of any of the Claims 1 to 14, further comprising a plurality of discrete uninsulated portions, the discrete uninsulated portions being spaced apart by one or more insulated portions of the second conductor (118).

## Patentansprüche

1. Medizinische Vorrichtung (100), umfassend:
eine Kernanordnung (105), die konfiguriert ist, um innerhalb eines physischen Lumens vorgeschoben zu werden, die Kernanordnung umfassend:
einen ersten Leiter (116), der einen proximalen Endabschnitt (116a) und einen distalen Endabschnitt (116b) aufweist, wobei der erste Leiter ein Lumen ausbildet;
einen zweiten Leiter (118), der mindestens teilweise innerhalb des Lumens des ersten Leiters eingerichtet ist, wobei der zweite Leiter ein erstes leitfähiges Material umfasst und einen proximalen Endabschnitt und einen distalen Endabschnitt aufweist, der sich distal zu dem distalen Endabschnitt des ersten Leiters erstreckt;
ein isolierendes Material (127), das über mindestens einen Abschnitt des zweiten Leiters eingerichtet ist, wobei das isolierende Material einen oder mehrere nicht isolierte Abschnitte des zweiten Leiters definiert; und
ein zweites leitfähiges Material (150, 152), das den zweiten Leiter entlang mindestens eines Teils des einen oder der mehreren nicht isolierten Abschnitte umgibt, wobei das zweite leitfähige Material eine höhere elektrische Leitfähigkeit als das erste leitfähige Material aufweist;
ein interventionelles Element (106), das mit dem distalen Endabschnitt des ersten Leiters gekoppelt ist, wobei das interventionelle Element elektrisch mit dem ersten Leiter gekoppelt ist, und wobei das interventionelle Element einen Körper (106b) umfasst, der aus einem dritten leitfähigen Material gebildet ist; und ein viertes leitfähiges Material, das über mindestens einen Abschnitt des dritten leitfähigen Materials des interventionellen Elements eingerichtet ist, wobei das vierte leitfähige Material eine höhere elektrische Leitfähigkeit als das dritte leitfähige Material aufweist.

2. Medizinische Vorrichtung (100) nach Anspruch 1, wobei das zweite leitfähige Material radial angrenzend an oder distal des interventionellen Elements (106) positioniert ist.

3. Medizinische Vorrichtung (100) nach Anspruch 1 oder Anspruch 2, wobei das dritte leitfähige Material Nitinol umfasst und/oder das erste leitfähige Material rostfreien Stahl umfasst.

4. Medizinische Vorrichtung (100) nach einem der Ansprüche 1 bis 3, wobei der distale Abschnitt des zweiten Leiters (118) eine distale Spitze (160) definiert, die eine vergrößerte radiale Abmessung aufweist.

5. Medizinische Vorrichtung (100) nach Anspruch 4, wobei der eine oder die mehreren nicht isolierten Abschnitte die distale Spitze (160) umfassen.

6. Medizinische Vorrichtung (100) nach einem der Ansprüche 1 bis 5, wobei eine Oberfläche, die durch das zweite leitfähige Material definiert ist, mindestens etwa 15 % einer Oberfläche beträgt, die durch das vierte leitfähige Material definiert ist.

7. Medizinische Vorrichtung (100) nach einem der Ansprüche 1 bis 6, wobei der erste und der zweite Leiter (116, 118) koaxial sind.

8. Medizinische Vorrichtung (100) nach einem der Ansprüche 1 bis 7, wobei die Kernanordnung (105) ein sich distal befindendes Verbindungselement (128) umfasst, das eine Öffnung darin aufweist, und wobei sich der zweite Leiter durch die Öffnung hindurch erstreckt.

9. Medizinische Vorrichtung (100) nach einem der Ansprüche 1 bis 8, wobei das interventionelle Element (106) ein Lumen definiert, und wobei sich der zweite Leiter (118) mindestens teilweise durch das Lumen hindurch erstreckt.

10. Medizinische Vorrichtung (100) nach Anspruch 9, wobei das zweite leitfähige Material innerhalb des Lumens eingerichtet ist.

11. Medizinische Vorrichtung (100) nach einem der Ansprüche 1 bis 10, wobei der erste Leiter und der zweite Leiter nicht verschiebbar miteinander gekoppelt sind.

12. Medizinische Vorrichtung (100) nach einem der Ansprüche 1 bis 11, wobei die Kernanordnung (105) so bemessen und konfiguriert ist, dass sie durch ein Blutgefäß zu einer Behandlungsstelle vorgeschoben werden kann.

13. Medizinische Vorrichtung (100) nach einem der Ansprüche 1 bis 12, wobei der distale Endabschnitt des zweiten Leiters (118) distal des distalen Endabschnitts des ersten Leiters (116) positioniert ist.

14. Medizinische Vorrichtung (100) nach einem der Ansprüche 1 bis 13, wobei das interventionelle Element (106) eine Vielzahl von Streben umfasst, die begrenzt sind, um eine Zellöffnung auszubilden, wobei der leitfähige Abschnitt radial angrenzend an die Zellöffnung positioniert ist.

15. Medizinische Vorrichtung (100) nach einem der Ansprüche 1 bis 14, ferner umfassend eine Vielzahl diskreter nicht isolierter Abschnitte, wobei die diskreten nicht isolierten Abschnitte durch einen oder mehrere isolierte Abschnitte des zweiten Leiters (118) voneinander beabstandet sind.

## Revendications

1. Dispositif médical (100) comprenant :
un ensemble noyau (105) configuré pour être avancé à l'intérieur d'une lumière corporelle, l'ensemble noyau comprenant :
un premier conducteur (116) ayant une partie d'extrémité proximale (116a) et une partie d'extrémité distale (116b), le premier conducteur formant une lumière ;
un second conducteur (118) au moins partiellement disposé à l'intérieur de la lumière du premier conducteur, le second conducteur comprenant un premier matériau conducteur et ayant une partie d'extrémité proximale et une partie d'extrémité distale s'étendant de manière distale à la partie d'extrémité distale du premier conducteur ;
un matériau isolant (127) disposé sur au moins une partie du second conducteur, le matériau isolant définissant une ou plusieurs parties non isolées du second conducteur ; et
un second matériau conducteur (150, 152) entourant le second conducteur le long d'au moins une partie de l'une ou plusieurs des parties non isolées, le second matériau conducteur ayant une conductivité électrique supérieure à celle du premier matériau conducteur ;
un élément d'intervention (106) couplé à la partie distale du premier conducteur, l'élément d'intervention étant couplé électriquement au premier conducteur, l'élément d'intervention comprenant un corps (106b) formé d'un troisième matériau conducteur ; et un quatrième matériau conducteur disposé sur au moins une partie du troisième matériau conducteur de l'élément interventionnel, le quatrième matériau conducteur ayant une conductivité électrique supérieure à celle du troisième matériau conducteur.

2. Dispositif médical (100) selon la revendication 1, dans lequel le second matériau conducteur est positionné radialement adjacent ou de manière distale à l'élément interventionnel (106).

3. Dispositif médical (100) selon l'une quelconque des revendications 1 ou 2, dans lequel le troisième matériau conducteur comprend du Nitinol, et/ou le premier matériau conducteur comprend de l'acier inoxydable.

4. Dispositif médical (100) selon l'une quelconque des revendications 1 à 3, dans lequel la partie distale du second conducteur (118) définit une pointe distale (160) ayant une dimension radiale élargie.

5. Dispositif médical (100) selon la revendication 4, dans lequel l'une ou plusieurs parties non isolées comprennent la pointe distale (160).

6. Dispositif médical (100) selon l'une quelconque des revendications 1 à 5, dans lequel une zone de surface définie par le second matériau conducteur est d'au moins 15 % environ d'une zone de surface définie par le quatrième matériau conducteur.

7. Dispositif médical (100) selon l'une quelconque des revendications 1 à 6, dans lequel les premier et second conducteurs (116, 118) sont coaxiaux.

8. Dispositif médical (100) selon l'une quelconque des revendications 1 à 7, dans lequel l'ensemble noyau (105) comprend un élément d'assemblage situé de manière distale (128) ayant une ouverture dans celui-ci, et dans lequel le second conducteur s'étend à travers l'ouverture.

9. Dispositif médical (100) selon l'une quelconque des revendications 1 à 8, dans lequel l'élément d'intervention (106) définit une lumière, et dans lequel le second conducteur (118) s'étend au moins partiellement à travers la lumière.

10. Dispositif médical (100) selon la revendication 9, dans lequel le second matériau conducteur est disposé à l'intérieur de la lumière.

11. Dispositif médical (100) selon l'une quelconque des revendications 1 à 10, dans lequel le premier conducteur et le second conducteur sont couplés ensemble de manière non coulissante.

12. Dispositif médical (100) selon l'une quelconque des revendications 1 à 11, dans lequel l'ensemble noyau (105) est dimensionné et configuré pour être avancé à travers un vaisseau sanguin jusqu'à un site de traitement.

13. Dispositif médical (100) selon l'une quelconque des revendications 1 à 12, dans lequel l'extrémité distale du second conducteur (118) est positionnée de manière distale par rapport à l'extrémité distale du premier conducteur (116).

14. Dispositif médical (100) selon l'une quelconque des revendications 1 à 13, dans lequel l'élément d'intervention (106) comprend une pluralité d'entretoises qui sont délimitées pour former une ouverture de cellule, la partie conductrice étant positionnée radialement adjacente à l'ouverture de cellule.

15. Dispositif médical (100) selon l'une quelconque des revendications 1 à 14, comprenant en outre une pluralité de portions discrètes non isolées, les portions discrètes non isolées étant espacées par une ou plusieurs portions isolées du second conducteur (118).
